# EUROPEAN PATENT SPECIFICATION

(11) **EP 3 903 686 B1**
(45) Date of publication and mention of the grant of the patent: **15.11.2023**
(21) Application number: 19905536.9
(22) Date of filing: 27.12.2019
(51) Int. Cl.: A61B 10/02, A61B 17/34, A61B 90/00

(54) **ROTATION PREVENTION BIOPSY NEEDLE**
BIOPSIENADEL MIT DREHSICHERUNG
AIGUILLE DE BIOPSIE ANTI-ROTATION

(30) Priority: 28.12.2018 CN 201822240603 U
(43) Date of publication of application: 03.11.2021
(73) Proprietor: Chamfond Biotech Co., Ltd, Nanjing, Jiangsu 210032 (CN)
(72) Inventor: LI, Yangjing, Nanjing, Jiangsu 210032 (CN); ZHANG, Jianhua, Nanjing, Jiangsu 210032 (CN)
(74) Representative: Gong, Jinping
(86) International application number: PCT/CN2019/129242
(87) International publication number: WO 2020/135726

(56) References cited:
- WO-A1-2015/074984
- CN-U- 203 736 237
- CN-Y- 2 899 718
- US-A1- 2012 136 277
- US-A1- 2017 196 559
- US-B1- 6 312 394

## Description

### Technical Field

The invention relates to a rotation preventing biopsy needle as specified in any of claims 1-13, and belongs to the technical field of medical instruments.

### Background Art

In clinical diagnosis, a biopsy needle is a kind of commonly used medical instrument. It can accurately position the nidus with a small wound, quickly complete sampling, and reduce the pain inflicted on a patient in an operation.

When the biopsy needle is used, firstly, an assembled biopsy needle is required to thread through skin tissues to reach a sampling position where it is inserted. The needle bar assembly is rotated to remove the needle bar, a syringe is connected through a luer, and the detection sample is sucked out through the syringe.

The sampling position of some biopsy needles is bone marrow so that the biopsy needle can be inserted into the sampling position to carry out sampling by using a strong force. Operating personnel usually drills the biopsy needle into the bone marrow by repeatedly rotating the biopsy needle with strong force. In this case, the needle bar handle may turn due to the friction between the handle of the needle bar and a palm, so that the active face of the needle bar is misaligned with that of the needle tube, leading to a weaker puncturing strength of the biopsy needle which may curl or even damage the needle tube and cause operation risks.

US 2012/136277 A1 describes a bone marrow aspiration assembly including an outer canula assembly, an inner canula assembly and a flexible seal.

### Summary of the Invention

The object of the invention: in order to overcome the defects in the prior art, the invention as specified in any of claims 1-13 provides a rotation preventing biopsy needle, which adopts the design of a limiting device and an unlocking device, can ensure that a needle bar of the biopsy needle does not turn in the puncturing process, and can be conveniently taken down when a needle bar assembly needs to be taken out.

Technical solutions: to solve the technical problem, the invention as specified in any of claims 1-13 provides a rotation preventing biopsy needle which includes a needle tube assembly and a needle bar assembly. The needle tube assembly includes a needle tube 5 and a needle tube handle 1, the needle bar assembly includes a needle bar 6 and a needle bar handle 2, the tail part of the needle tube 5 is connected with the needle tube handle 1, the top part of the needle tube handle 1 is provided with a luer 103, the needle bar 6 penetrates through the needle tube handle 1 and the needle tube 5 through the luer 103, the tail part of the needle bar 6 has the needle bar handle 2, a first limiting device is arranged on the needle tube handle 1, the first limiting device includes a rotary buckle 101 and a limiting buckle 102 which are located on two sides of the luer 103, a supporting part 101-1 of the rotary buckle 101 is supported on one side of the luer 103, and the supporting part 101-1 extends upwards to form a hook part 101-2 which is arranged at a right angle with the supporting part 101-1.

Still as defined by the appended claim set, the needle bar handle 2 is arranged with a second limiting device, the second limiting device comprising a rotary groove 201 mated with the rotary buckle 101, and the rotary groove 201 enables the hook part 101-2 to be rotated in and locked to limit the axial movement of the needle bar handle; the second limiting device further includes a guide groove 202 mated with the limiting buckle 102, one end of the guide groove with a small diameter is connected with a limiting groove 203, and the guide groove 202 is a section of a notch with a linearly reduced diameter. The limiting buckle 101 is inserted into one end of the guide groove 202 with a large diameter at the initial stage. The limiting buckle 101 slides along the guide groove 202 towards one end with a small diameter to enter the limiting groove 203 along with the rotation of the needle bar handle. As the diameter of the guide groove becomes smaller, the limiting buckle 102 is elastically deformed in the radial direction. When the limiting buckle 102 rotates to the end of the guide groove 202, the limiting buckle 102 springs back into place and enters the limiting groove 203. At this time, the axial rotation of the needle bar handle 2 is limited.

Still as defined by the appended claim set, an unlocking device is further arranged on the needle bar handle, and the unlocking device includes an unlocking button 301 located on one side of the limiting buckle 102. The limiting buckle 102, when in the locked state, is located in the limiting groove. When unlocking is needed, the unlocking button 301 inclines towards the limiting buckle 102, making the limiting buckle deform so that the limiting buckle and the limiting groove are staggered. At such time, the limiting effect of the limiting buckle disappears, so that the needle bar handle can be rotated.

Still as defined by the appended claim set, the needle bar assembly further includes a needle bar handle cover 3. One group of grooves are arranged in the circumferential direction of the needle bar handle cover 3 and can play an antiskid role.

Still as defined by the appended claim set, needle tube 5 has a hollow structure, and the end thereof has an inclined plane puncturing needle head 501.

Further, the needle bar 6 has a solid core structure, the end thereof coinciding with the inclined plane puncturing needle head 501. A puncturing edge is formed when the biopsy needle enters a biopsy position.

Still as defined by the appended claim set, needle tube 5 penetrates through an adjusting handle 4 to be connected with the needle tube handle 1. Operating personnel can rotate a nut on the adjusting handle 4 according to the requirements of different puncturing depths so that the adjusting handle moves back and forth, the exposed length of the needle tube 5 is adjusted, and the operation is simple and convenient.

The beneficial effects are as follows: (1) the biopsy needle handle rotation preventing structure of the invention is simple in structure, and as only a few simple changes need to be made on the original biopsy needle structure, the changing cost is low; (2) according to the invention, rotations caused by mistaken touching of the handle in the operation process can be effectively prevented, the effectiveness of instruments is ensured, and the operation risk is reduced; (3) the unlocking device of the invention is easy to operate by hands, and the user experience is good.

### Brief Description of the Drawings

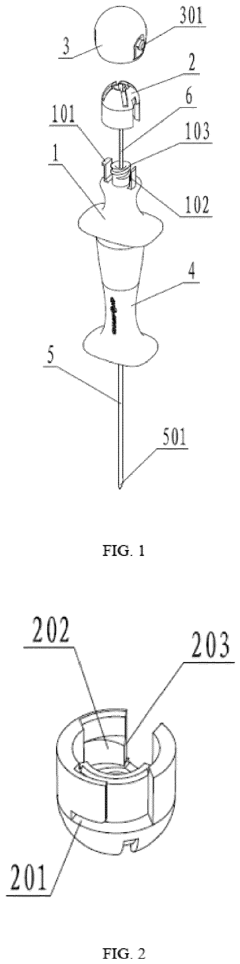

Fig. 1 is a schematic view showing a structure of an embodiment of the present invention.
Fig. 2 is a schematic view showing a structure of a needle bar handle of an embodiment of the present invention.

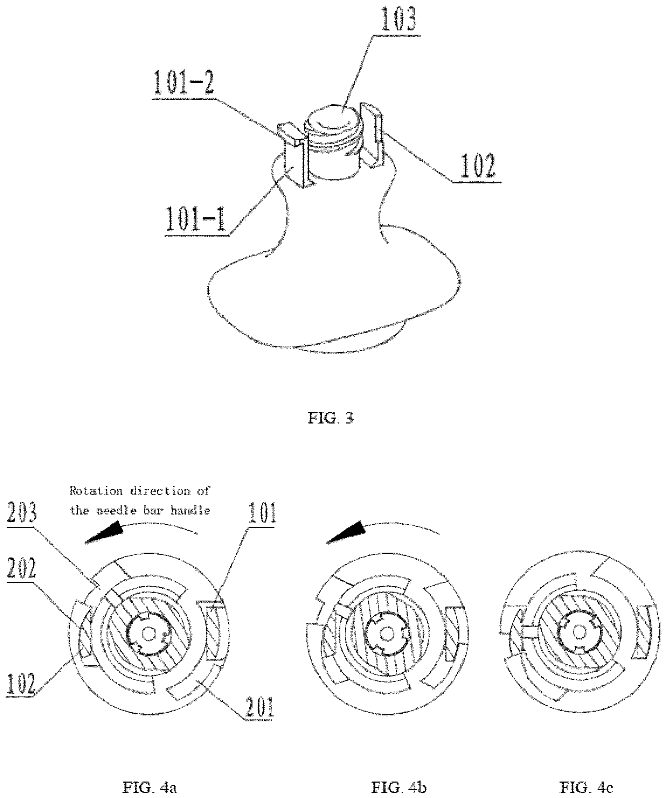

Fig. 3 is a schematic view showing a structure of a needle tube handle of an embodiment of the present invention.
Fig. 4a is a schematic view showing a rotary buckle and a limiting buckle in an initial stage of an embodiment of the present invention.
Fig. 4b is a schematic view of a rotary buckle and a limiting buckle in a rotary stage of an embodiment of the present invention.
Fig. 4c is a schematic view showing a rotary buckle and a limiting buckle in a meshing stage of an embodiment of the present invention.

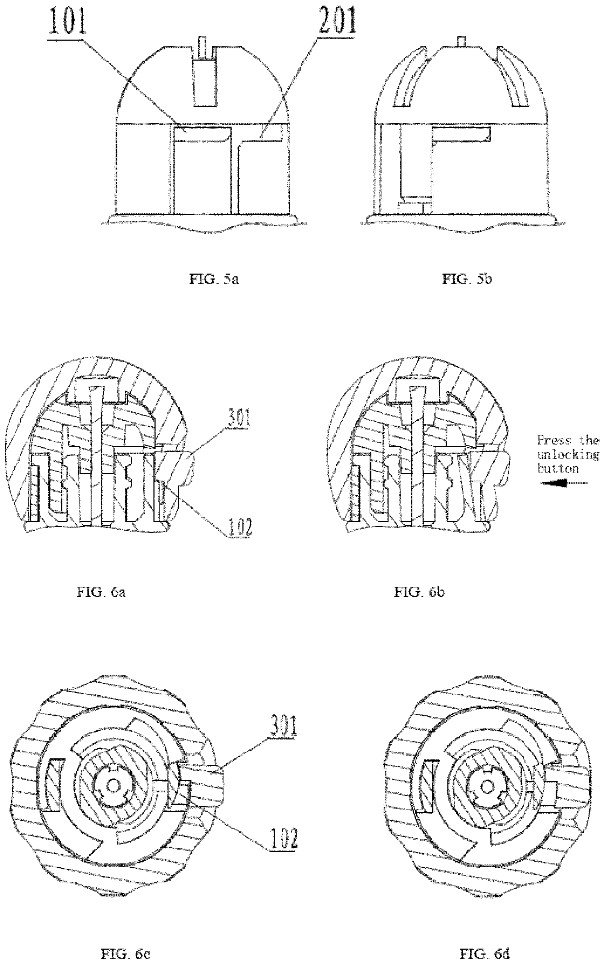

Fig. 5a is another schematic view showing a rotary buckle in an initial stage of an embodiment of the present invention.
Fig. 5b is another schematic view showing a rotary buckle in a completion stage of an embodiment of the present invention.
Fig. 6a is a schematic view showing a limiting buckle in a locked state of an embodiment of the present invention.
Fig. 6b is a schematic view showing a limiting buckle in an unlocked state of an embodiment of the present invention.
Fig. 6c is another schematic view showing a limiting buckle in a locked state of an embodiment of the present invention.
Fig. 6d is another schematic view showing a limiting buckle in an unlocked state of an embodiment of the present invention.

### Detailed Description of the Invention

The present invention will be further described below in conjunction with the drawings.

### Embodiment

The invention as specified in any of claims 1-13 provides a rotation preventing biopsy needle which includes a needle tube assembly and a needle bar assembly. The needle tube assembly includes a needle tube 5 and a needle tube handle 1, the needle bar assembly includes a needle bar 6 and a needle bar handle 2, the tail part of the needle tube 5 is connected with the needle tube handle 1, the top part of the needle tube handle 1 is provided with a luer 103, the needle bar 6 penetrates through the needle tube handle 1 and the needle tube 5 through the luer 103, the tail part of the needle bar 6 has the needle bar handle 2, a first limiting device is arranged on the needle tube handle 1, the first limiting device includes a rotary buckle 101 and a limiting buckle 102 which are located on two sides of the luer 103, a supporting part 101-1 of the rotary buckle 101 is supported on one side of the luer 103, and the supporting part 101-1 extends upwards to form a hook part 101-2 which is arranged at a right angle with the supporting part 101-1.

The hook part 101-2 is rotated in and locked to limit the axial movement of the needle bar handle; the second limiting device further includes a guide groove 202 mated with the limiting buckle 102, one end of the guide groove with a small diameter is connected with a limiting groove 203, and the guide groove 202 is a section of a notch with a linearly reduced diameter. The limiting buckle 101 is inserted into one end of the guide groove 202 with a large diameter at the initial stage. The limiting buckle 101 slides along the guide groove 202 towards one end with a small diameter to enter the limiting groove 203 along with the rotation of the needle bar handle. As the diameter of the guide groove becomes smaller, the limiting buckle 102 is elastically deformed in the radial direction. When the limiting buckle 102 rotates to the end of the guide groove 202, the limiting buckle 102 springs back into place and enters the limiting groove 203. At this time, the axial rotation of the needle bar handle 2 is limited.

An unlocking device is further arranged on the needle bar handle, and the unlocking device includes an unlocking button 301 located on one side of the limiting buckle 102. The limiting buckle 102, when in the locked state, is located in the limiting groove. When unlocking is needed, the unlocking button 301 inclines towards the limiting buckle 102, making the limiting buckle deform so that the limiting buckle and the limiting groove are staggered. At such time, the limiting effect of the limiting buckle disappears, so that the needle bar handle can be rotated. The needle bar assembly further includes a needle bar handle cover 3. One group of grooves are arranged in the circumferential direction of the needle bar handle cover 3 and can play an antiskid role. The needle tube 5 has a hollow structure, the end thereof having an inclined plane puncturing needle head 501. The needle bar 6 has a solid core structure, the end thereof coinciding with the inclined plane puncturing needle head 501. A puncturing edge is formed when the biopsy needle enters a biopsy position. The needle tube 5 penetrates through the adjusting handle 4 to be connected with the needle tube handle 1.

When the invention is applied in practice, operating personnel thread the assembled biopsy needle through skin tissues to reach a sampling position. At such time, the biopsy needle is drilled into the sampling position by great rotating force, and the needle bar assembly does not turn and loosen due to the rotation action of a hand. After the biopsy needle is completely inserted into the sampling position, a thumb presses the unlocking button and then rotates the needle bar handle cover. Now, the needle bar assembly can be removed. The invention provides a good user experience, fundamentally does not change the original user operation habit, and is not difficult to operate by hands; moreover, the rotation of the needle bar of the biopsy needle in the puncturing process can be prevented so that the risk of operations is reduced, and the use of the instrument is more reliable.

## Claims

1. A rotation preventing biopsy needle, comprising a needle tube assembly and a needle bar assembly, wherein the needle tube assembly comprises a needle tube (5) and a needle tube handle (1), the needle bar assembly comprises a needle bar (6) and a needle bar handle (2), a tail part of the needle tube is connected with the needle tube handle (1), and a tail part of the needle bar (6) is connected with the needle bar handle (2), **characterized in that**:
the needle tube handle (1) is arranged with a first limiting device, the first limiting device comprising a limiting buckle (102);
the needle bar handle (2) is arranged with a second limiting device, the second limiting device comprising a guide groove (202) configured to mate with the limiting buckle (102), one end of the guide groove with a smaller diameter is a limiting groove (203), whereby the limiting buckle (102) is configured to be inserted into one end of the guide groove (202) with a larger diameter in an initial stage, along with a rotation of the needle bar handle (2), whereby an outer side surface of the limiting buckle (102) is configured to rotate along an inner side surface fitting the guide groove (202) and is stressed to deform towards an inner side, the limiting buckle further configured such that when the limiting buckle (102) completely enters the limiting groove (203), the limiting buckle (102) recovers its shape and then is meshed to limit an axial rotation of the needle bar handle (2).

2. The rotation preventing biopsy needle according to claim 1, wherein the guide groove (202) is a section of a notch with a linearly reduced diameter.

3. The rotation preventing biopsy needle according to claim 1, wherein the first limiting device further comprises a rotary buckle (101), whereby a supporting part (101-1) of the rotary buckle (101) is connected with the needle tube handle (1), and the supporting part (101-1) extends upwards to form a hook part (101-2); whereby the second limiting device further comprises a rotary groove (201) configured to mate with the rotary buckle (101), the rotary groove (201) enabling the hook part (101-2) to be rotated in and locked so that an axial movement of the needle bar handle is limited.

4. The rotation preventing biopsy needle according to claim 3, wherein the supporting part (101-1) extends upwards to form a hook part (101-2) arranged at a right angle with the supporting part (101-1).

5. The rotation preventing biopsy needle according to claim 3, whereby a luer connector (103) is provided at a top of the needle tube handle (1), and the needle bar (6) penetrates through the needle tube handle (1) and the needle tube (5) through the luer connector (103).

6. The rotation preventing biopsy needle according to claim 5, whereby the limiting buckle (102) and the rotary buckle (101) are located on two sides of the luer connector (103).

7. The rotation preventing biopsy needle according to claim 1, whereby it further comprises: an unlocking device, wherein the unlocking device comprises an unlocking button (301) located on an outer side of the limiting buckle (102), the limiting buckle (102) being located in the limiting groove (203) in a locked state, wherein the unlocking button (301) is configured to incline inclines towards the limiting buckle (102) for the purpose of unlocking, and the limiting buckle (102) is configured to deform, disengaging the limiting buckle (102) and the limiting groove (203), such that a limiting effect of the limiting buckle (102) disappears so that the needle bar handle (2) can be rotated.

8. The rotation preventing biopsy needle according to claim 1, wherein a top part of the needle tube handle (1) is provided with a luer connector (103), wherein the first limiting device comprises a rotary buckle (101) located on one side of the luer (103) and the limiting buckle (102) being opposite to the rotary buckle (101), whereby a supporting part (101-1) of the rotary buckle (101) is supported on one side of the luer (103), and the supporting part (101-1) extends upwards to form a hook part (101-2) which is arranged at a right angle with the supporting part (101-1); the second limiting device comprising a rotary groove (201) configured to mate with the rotary buckle (101), and wherein the rotary groove (201) is configured to enable the hook part (101-2) to be rotated in and locked so that an axial movement of the needle bar handle is limited.

9. The rotation preventing biopsy needle according to claim 8, whereby the needle bar handle is further arranged with an unlocking device, wherein the unlocking device comprises an unlocking button (301) located on an outer side of the limiting buckle (102), the limiting buckle (102) being located in the limiting groove (203) in a locked state, wherein the unlocking button (301) is configured to incline towards the limiting buckle (102) for the purpose of unlocking, and the limiting buckle (102) is configured to deform, disengaging the limiting buckle (102) and the limiting groove (203), such that the needle bar handle can be rotated.

10. The rotation preventing biopsy needle according to claim 8, whereby the needle bar assembly further comprises a needle bar handle cover (3), and one group of grooves are arranged in the circumferential direction of the needle bar handle cover (3).

11. The rotation preventing biopsy needle according to claim 8, whereby the needle tube (5) has a hollow structure, and an end thereof has an inclined plane puncturing needle head (501).

12. The rotation preventing biopsy needle according to claim 11, whereby the needle bar (6) has a solid core structure, and an end thereof has an inclined plane at a same angle with the puncturing needle head (501) and is configured such that it can be kept flush with the puncturing needle head to form a puncturing edge.

13. The rotation preventing biopsy needle according to claim 8, whereby the needle tube (5) penetrates through an adjusting handle (4) and is connected with the needle tube handle (1).

## Patentansprüche

1. Drehung verhindernde Biopsienadel, umfassend eine Nadelrohrbaugruppe und eine Nadelstangenbaugruppe, wobei die Nadelrohrbaugruppe ein Nadelrohr (5) und einen Nadelrohrgriff (1) umfasst, wobei die Nadelstangenbaugruppe eine Nadelstange (6) und einen Nadelstangengriff (2) umfasst, wobei ein hinterer Teil des Nadelrohrs mit dem Nadelrohrgriff (1) verbunden ist, und ein hinterer Teil der Nadelstange (6) mit dem Nadelstangengriff (2) verbunden ist, **dadurch gekennzeichnet,**
**dass** der Nadelrohrgriff (1) mit einer ersten Begrenzungsvorrichtung ausgestattet ist, wobei die erste Begrenzungsvorrichtung eine Begrenzungsschnalle (102) umfasst;
**dass** der Nadelstangengriff (2) mit einer zweiten Begrenzungsvorrichtung ausgestattet ist, wobei die zweite Begrenzungsvorrichtung eine Führungsnut (202) umfasst, die dazu ausgebildet ist, mit der Begrenzungsschnalle (102) zusammenzupassen, wobei ein Ende der Führungsnut mit einem kleineren Durchmesser eine Begrenzungsnut (203) ist, wobei die Begrenzungsschnalle (102) dazu ausgebildet ist, in einem Anfangsstadium bei einer Drehung des Nadelstangengriffs (2) in ein Ende der Führungsnut (202) mit einem größeren Durchmesser eingeführt zu werden, wobei eine äußere Seitenfläche der Begrenzungsschnalle (102) dazu ausgebildet ist, entlang einer inneren Seitenfläche der Führungsnut (202) zu drehen, und zur Verformung in Richtung einer Innenseite beansprucht wird, wobei die Begrenzungsschnalle weiterhin so ausgebildet ist, dass wenn die Begrenzungsschnalle (102) vollständig in die Begrenzungsnut (203) eindringt, die Begrenzungsschnalle (102) ihre Form wieder annimmt und greift dann ein, um eine axiale Drehung des Nadelstangengriffs (2) zu begrenzen.

2. Drehung verhindernde Biopsienadel nach Anspruch 1, wobei die Führungsnut (202) ein Abschnitt einer Kerbe mit einem linear verringerten Durchmesser ist.

3. Drehung verhindernde Biopsienadel nach Anspruch 1, wobei die erste Begrenzungsvorrichtung weiterhin eine Drehschnalle (101) umfasst, wobei ein Stützteil (101-1) der Drehschnalle (101) mit dem Nadelrohrgriff (1) verbunden ist, und der Stützteil (101-1) sich nach oben erstreckt, um einen Hakenteil (101-2) zu bilden; wobei die zweite Begrenzungsvorrichtung weiterhin eine Drehnut (201) umfasst, die dazu ausgebildet ist, mit der Drehschnalle (101) zusammenzupassen, wobei die Drehnut (201) das Eindrehen und Verriegeln des Hakenteils (101-2) ermöglicht, so dass eine axiale Bewegung des Nadelstangengriffs begrenzt ist.

4. Drehung verhindernde Biopsienadel nach Anspruch 3, wobei sich der Stützteil (101-1) nach oben erstreckt, um einen Hakenteil (101-2) zu bilden, der im rechten Winkel zum Stützteil (101-1) angeordnet ist.

5. Drehung verhindernde Biopsienadel nach Anspruch 3, wobei an der Oberseite des Nadelrohrgriffs (1) ein Luer-Anschluss (103) vorgesehen ist, und die Nadelstange (6) durch den Nadelrohrgriff (1) und das Nadelrohr (5) durch den Luer-Anschluss (103) dringt.

6. Drehung verhindernde Biopsienadel nach Anspruch 5, wobei die Begrenzungsschnalle (102) und die Drehschnalle (101) auf zwei Seiten des Luer-Anschlusses (103) angeordnet sind.

7. Drehung verhindernde Biopsienadel nach Anspruch 1, wobei sie weiterhin umfasst: eine Entriegelungsvorrichtung, wobei die Entriegelungsvorrichtung einen Entriegelungsknopf (301) umfasst, der sich an einer Außenseite der Begrenzungsschnalle (102) befindet, wobei die Begrenzungsschnalle (102) in einem verriegelten Zustand in der Begrenzungsnut (203) angeordnet ist, wobei der Entriegelungsknopf (301) dazu ausgebildet ist, in Richtung der Begrenzungsschnalle (102) zur Entriegelung zu neigen, und die Begrenzungsschnalle (102) zur Verformung ausgebildet ist, um die Begrenzungsschnalle (102) und die Begrenzungsnut (203) zu lösen, so dass eine Begrenzungswirkung der Begrenzungsschnalle (102) verschwindet, so dass der Nadelstangengriff (2) gedreht werden kann.

8. Drehung verhindernde Biopsienadel nach Anspruch 1, wobei ein oberer Teil des Nadelrohrgriffs (1) mit einem Luer-Anschluss (103) versehen ist, wobei die erste Begrenzungsvorrichtung eine auf einer Seite des Luer (103) angeordnete Drehschnalle (101) umfasst, und die Begrenzungsschnalle (102) der Drehschnalle (101) gegenüberliegt, wobei ein Stützteil (101-1) der Drehschnalle (101) auf einer Seite des Luer (103) abgestützt ist, und der Stützteil (101-1) sich nach oben erstreckt, um einen Hakenteil (101-2) zu bilden, der im rechten Winkel zum Stützteil (101-1) angeordnet ist;
wobei die zweite Begrenzungsvorrichtung eine Drehnut (201) umfasst, die dazu ausgebildet ist, mit der Drehschnalle (101) zusammenzupassen, und wobei die Drehnut (201) ausgebildet ist, das Eindrehen und Verriegeln des Hakenteils (101-2) zu ermöglichen, so dass eine axiale Bewegung des Nadelstangengriffs begrenzt ist.

9. Drehung verhindernde Biopsienadel nach Anspruch 8, wobei der Nadelstangengriff weiterhin mit einer Entriegelungsvorrichtung ausgestattet ist, wobei die Entriegelungsvorrichtung einen Entriegelungsknopf (301) umfasst, der sich an einer Außenseite der Begrenzungsschnalle (102) befindet, wobei die Begrenzungsschnalle (102) in einem verriegelten Zustand in der Begrenzungsnut (203) angeordnet ist, wobei der Entriegelungsknopf (301) dazu ausgebildet ist, in Richtung der Begrenzungsschnalle (102) zur Entriegelung zu neigen, und die Begrenzungsschnalle (102) zur Verformung ausgebildet ist, um die Begrenzungsschnalle (102) und die Begrenzungsnut (203) zu lösen, so dass der Nadelstangengriff gedreht werden kann.

10. Drehung verhindernde Biopsienadel nach Anspruch 8, wobei die Nadelstangenbaugruppe weiterhin eine Nadelstangengriffabdeckung (3) umfasst und eine Gruppe von Nuten in Umfangsrichtung der Nadelstangengriffabdeckung (3) angeordnet ist.

11. Drehung verhindernde Biopsienadel nach Anspruch 8, wobei das Nadelrohr (5) eine hohle Struktur aufweist, und ein Ende davon einen geneigten ebenen Punktionsnadelkopf (501) aufweist.

12. Drehung verhindernde Biopsienadel nach Anspruch 11, wobei die Nadelstange (6) eine massive Kernstruktur aufweist, und ein Ende davon eine geneigte Ebene im gleichen Winkel mit dem Punktionsnadelkopf (501) aufweist und so ausgebildet ist dass es bündig mit dem Punktionsnadelkopf gehalten werden kann, um eine Punktionskante zu bilden.

13. Drehung verhindernde Biopsienadel nach Anspruch 8, wobei das Nadelrohr (5) durch einen Einstellgriff (4) hindurchdringt und mit dem Nadelrohrgriff (1) verbunden ist.

## Revendications

1. Une aiguille de biopsie empêchant la rotation, comprenant un ensemble tube à aiguille et un ensemble barre à aiguille, dans lequel l'ensemble tube à aiguille comprend un tube à aiguille (5) et une poignée de tube à aiguille (1), l'ensemble barre à aiguille comprend une barre à aiguille (6) et une poignée de barre à aiguille (2), une partie arrière du tube à aiguille est reliée à la poignée de tube à aiguille (1), et une partie arrière de la barre à aiguille (6) est reliée à la poignée de barre à aiguille (2), **caractérisé en ce que** :
la poignée de tube à aiguille (1) est équipée d'un premier dispositif de limitation, le premier dispositif de limitation comprenant une boucle de limitation (102) ;
la poignée de barre à aiguille (2) est équipée d'un deuxième dispositif de limitation, le deuxième dispositif de limitation comprenant une rainure de guidage (202) configurée pour s'accoupler avec la boucle de limitation (102), une extrémité de la rainure de guidage d'un diamètre inférieur est une rainure de limitation (203), dans lequel la boucle de limitation (102) étant configurée pour être insérée dans une extrémité de la rainure de guidage (202) d'un diamètre supérieur dans une phase initiale, avec la rotation de la poignée de barre à aiguille (2), dans lequel une surface latérale extérieure de la boucle de limitation (102) étant configurée pour s'adapter le long d'une surface latérale intérieure correspondant à la rainure de guidage (202) et étant contrainte de se déformer vers un côté intérieur, la boucle de limitation est en outre configurée de telle sorte que lorsque la boucle de limitation (102) pénètre complètement dans la rainure de limitation (203), la boucle de limitation (102) reprend sa forme puis s'engrène pour limiter une rotation axiale de la poignée de barre à aiguille (2).

2. L'aiguille de biopsie empêchant la rotation selon la revendication 1, dans laquelle la rainure de guidage (202) est une section d'une encoche avec un diamètre linéairement réduit.

3. L'aiguille de biopsie empêchant la rotation selon la revendication 1, dans laquelle le premier dispositif de limitation comprend en outre une boucle rotative (101), dans laquelle une partie de support (101-1) de la boucle rotative (101) est reliée à la poignée de tube à aiguille (1), et la partie de support (101-1) s'étend vers le haut pour former une partie crochet (101-2) ;
dans lequel le second dispositif de limitation comprenant en outre une rainure rotative (201) configurée pour s'accoupler avec la boucle rotative (101), la rainure rotative (201) permettant à la partie crochet (101-2) d'être tournée et verrouillée de sorte qu'un mouvement axial de la poignée de barre à aiguille est limitée.

4. L'aiguille de biopsie empêchant la rotation selon la revendication 3, dans laquelle la partie de support (101-1) s'étend vers le haut pour former une partie en crochet (101-2) disposée à angle droit avec la partie de support (101-1).

5. L'aiguille de biopsie empêchant la rotation selon la revendication 3, dans laquelle un connecteur Luer (103) est prévu au sommet de la poignée de tube d'aiguille (1), et la barre à aiguille (6) pénètre à travers la poignée de tube à aiguille (1) et le tube à aiguille (5) à travers le connecteur Luer (103).

6. L'aiguille de biopsie empêchant la rotation selon la revendication 5, dans laquelle la boucle de limitation (102) et la boucle rotative (101) se trouve sur deux côtés du connecteur Luer (103).

7. L'aiguille de biopsie empêchant la rotation selon la revendication 1, comprenant en outre: un dispositif de déverrouillage, dans lequel le dispositif de déverrouillage comprend un bouton de déverrouillage (301) se trouve sur un côté extérieur de la boucle de limitation (102), la boucle de limitation (102) se trouve dans la rainure de limitation (203) dans un état verrouillé, dans lequel le bouton de déverrouillage (301) est configuré pour s'incliner vers la boucle de limitation (102) dans le but de déverrouiller, et la boucle de limitation (102) est configurée pour se déformer, désengageant la boucle de limitation (102) et la rainure de limitation (203), de sorte qu'un effet de limitation de la boucle de limitation (102) disparaisse de sorte que la poignée de barre à aiguille (2) puisse être tourné.

8. L'aiguille de biopsie empêchant la rotation selon la revendication 1, dans laquelle une partie supérieure de la poignée du tube à aiguille (1) est pourvue d'un connecteur Luer (103), dans lequel le premier dispositif de limitation comprend une boucle rotative (101) se trouve sur un côté du Luer (103) et la boucle de limitation (102) étant opposée à la boucle rotative (101), dans lequel une partie de support (101-1) de la boucle rotative (101) est supportée sur un côté du Luer (103), et la partie de support (101-1) s'étend vers le haut pour former une partie crochet (101-2) qui est disposé à angle droit avec la partie de support (101-1);
le second dispositif de limitation comprenant une rainure rotative (201) configurée pour s'accoupler avec la boucle rotative (101), et dans laquelle la rainure rotative (201) est configurée pour permettre à la partie crochet (101-2) d'être tournée et verrouillée de sorte qu'un mouvement axial de la poignée de barre à aiguille est limitée.

9. L'aiguille de biopsie empêchant la rotation selon la revendication 8, dans laquelle la poignée de barre à aiguille est en outre disposés d'un dispositif de déverrouillage, dans lequel le dispositif de déverrouillage comprend un bouton de déverrouillage (301) se trouve sur un côté extérieur de la boucle de limitation (102), la boucle de limitation (102) se trouve dans la rainure de limitation (203) dans un état verrouillé, dans laquelle le bouton de déverrouillage (301) est configuré pour s'incliner vers la boucle de limitation (102) dans le but de déverrouiller, et la boucle de limitation (102) est configurée pour se déformer, désengageant la boucle de limitation (102) et la rainure de limitation (203), de sorte que la poignée de barre à aiguille puisse être tournée.

10. L'aiguille de biopsie empêchant la rotation selon la revendication 8, dans laquelle l'ensemble de barre à aiguille comprend en outre un couvercle de poignée de barre à aiguille (3), et un groupe de rainures est disposé dans la direction circonférentielle du couvercle de poignée de barre à aiguille (3).

11. L'aiguille de biopsie empêchant la rotation selon la revendication 8, dans laquelle le tube à aiguille (5) a une structure creuse, et une extrémité de celui-ci a une tête à aiguille de perforation (501) plane inclinée .

12. L'aiguille de biopsie empêchant la rotation selon la revendication 11, dans laquelle la barre à aiguille (6) a une structure de noyau solide, et une extrémité de celle-ci a un plan incliné faisant un même angle avec la tête à aiguille de perforation (501) et est configurée de telle sorte qu'elle puisse être maintenu au ras de la tête à aiguille de perforation pour former un bord de perforation.

13. L'aiguille de biopsie empêchant la rotation selon la revendication 8, dans laquelle le tube à aiguille (5) pénètre à travers une poignée de réglage (4) et est relié à la poignée de tube à aiguille (1).
